# EUROPEAN PATENT SPECIFICATION

(11) **EP 3 789 065 B1**
(45) Date of publication and mention of the grant of the patent: **22.06.2022**
(21) Application number: 19196169.7
(22) Date of filing: 09.09.2019
(51) Int. Cl.: A61M 15/00, A61B 5/087

(54) **AN ELECTRONIC SYSTEM COMPRISING A DRY POWDER INHALER AND AN EXTERNAL DEVICE**
EIN ELEKTRONISCHES SYSTEM UMFASSEND EINEN TROCKENPULVERINHALATOR UND EIN EXTERNES GERÄT
UN SYSTÈME ÉLECTRONIQUE COMPRENANT UN INHALATEUR DE POUDRE SÈCHE ET UN DISPOSITIF EXTERNE

(43) Date of publication of application: 10.03.2021
(73) Proprietor: Presspart GmbH & Co. KG, 34431 Marsberg (DE)
(72) Inventor: Jung, Benjamin, 50668 Köln (DE); Sule, Sunita, 401107 Maharashtra (IN); Ebert, Anselm, 97204 Höchberg (DE); Sule, Ameet, 401107 Maharashtra (IN)
(74) Representative: Uexküll & Stolberg

(56) References cited:
- EP-A1- 3 111 978
- EP-A1- 3 199 193
- EP-A1- 3 248 641
- WO-A1-2012/123448
- WO-A1-2015/136529
- WO-A1-2017/068171
- WO-A1-2017/201463
- WO-A1-2018/160073
- US-A1- 2015 196 724

## Description

### FIELD OF THE INVENTION

The present invention relates to an electronic system comprising an inhaler device for dispensing a medicament formulation in dry powder form and an external device which is configured to receive data communicated from the inhaler device. The invention also relates to a method of using the electronic system for spirometry.

### BACKGROUND OF THE INVENTION

Inhaler devices such as inhalers are commonly used to deliver drugs into the lung of a patient in need thereof. Different types of inhalers have been developed and are available on the market, amongst which dry powder inhalers (DPIs) are attractive in the treatment of various respiratory problems such as asthma, bronchitis or chronic obstructive pulmonary disease (COPD). In DPIs, the dose of medicament is present in dry powder form and usually pre-packed in containers such as a capsule. Blister-based dry powder inhalers as well as reservoir inhalers are also known.

In capsule-based DPIs, a capsule is placed into a capsule chamber of the inhaler and opened, e.g. by piercing the capsule at its ends, before inhalation. Subsequently, the patient inhales through a nose- or mouthpiece whereupon an inhalation airflow is generated within the inhaler. The medicament in dry powder form is released from the capsule, and entrained into the inhalation air flow and inhaled by the patient.

Nowadays smart inhalers are becoming more and more attractive. These inhalers comprise one or more sensors which allow detection of inhalation of a medicament. Moreover, these inhalers comprise a transmitter by which data is communicated from the inhaler to an external device. For example smart inhalers allow tracking of the use of the inhaler over a long term period. Physicians may then check how often the inhaler has been used.

An electronic system comprising an inhaler and an external device is for example known from EP 3 199 193 A1. This document relates to a system comprising an inhaler adapted to aerosolize a formulation contained in a capsule and a computing device external of the inhaler. The inhaler comprises a sensor, i.e. a microphone in order to sense the sound of the capsule impacting on the inhaler and a data-receiving-transmitting means to receive and transmit data from and to the external computing device. By measuring the impacts on the capsule movement inside the inhaler the technology described in this document retrieves information regarding the flow characteristics in the inhaler during inhalation.

Moreover, the company NYPRO published an article "Smart drug delivery: Pragmatic approach reduces risk, increases innovations & patient adherence" in 2017. This article relates to a smart drug delivery system called Ruby which is a dry powder inhaler providing the powdered medication in blisters. The inhaler comprises a pressure sensor in order to measure inhalation performance by a patient. Moreover, the inhaler comprises a hall sensor in order to track when the inhaler is triggered.

WO 2012/123448 A1 relates to an inhaler, specifically a capsule based inhaler

EP 3111 978 A1 relates to an inhaler as well as the use of the inhaler for inhalation therapy.

WO 2018/160073 A1 relates to methods, devices and systems for monitoring adherence to medication regimes for inhalers.

WO 2015/136529 A1 relates to methods and systems for controlled delivery of dry powder.

US 2015/196724 A1 relates to an interactive apparatus which can be used alone and/or combined with an inhalation simulation system which can record and reproduce, or simulate a patient inhalation effort.

WO 2017/201463 A1 also relates to interactive apparatuses and methods for recording, transferring and displaying physical measurements based on physiological conditions generated by a subject during an inhalation maneuver in real-time.

EP 3 248 641 A1 relates to an inhaler device for delivering a dose of medicament in dry powder form from a container to a patient in need thereof.

The effect of an inhaled pharmaceutical formulation is heavily dependent on, amongst others, the ability of the patient to inhale the right amount of the medicament formulation. It has been found that a lot of patients living with asthma do not use their inhaler adequately and thus do not receive the correct amount of medication upon inhalation.

It is therefore an object of the present invention to provide a cost effective and highly reliable electronic system which allows improved detection of correct inhalation of a medicament formulation by a patient.

### SUMMARY OF THE INVENTION

This object is achieved by an electronic system comprising the features of claim 1. Preferred embodiments are set out in the dependent claims.

According to the present invention the electronic system comprises an inhaler device for dispensing a medicament formulation in dry powder form and an external device which is configured to receive data communicated from the inhaler device. The inhaler device comprises a housing with an air inlet duct as well as a medicament cavity which is connected to the air inlet duct and which is configured to hold at least a dose of the medicament formulation. Moreover, the inhaler device comprises a mouthpiece having an outlet duct which is connected to the medicament cavity such that a patient upon inhalation through the outlet duct causes an inhalation flow directed from the air inlet duct through the medicament cavity towards the outlet duct. The inhaler device further comprises a primary sensor device which is configured to generate sensor signals and a wireless transmitting unit for communicating data related to the sensor signals. The electronic system further comprises a processing unit for processing the sensor signals and/or the communicated data.

According to the present invention the processing unit is configured to analyze the content of the medicament cavity based on the generated sensor signals of the primary sensor device.

In an embodiment of the invention the primary sensor device further comprises one or more of an optical sensor, a flow rate sensor, a touch sensor, a proximity sensor or any combination thereof. Preferably the primary sensor device comprises an optical sensor and/or a flow rate sensor.

In connection with the present invention an "optical sensor" is understood as a sensor which transmits and/or receives light. Hence, one or more optical sensors in combination, based on the amount of received light compared to the amount of transmitted light, are able to generate a corresponding sensor signal.

The optical sensor is preferably configured to transmit light through the medicament cavity in order to detect any content therein. Additionally or alternatively a flow rate sensor may be used in order to measure the flow rate of the inhalation flow. A flow rate sensor may be for example a pressure sensor such as a differential pressure sensor.

In connection with the present invention it has been found that the measured flow rate of the inhalation flow during inhalation by a patient differs if for example a dose of medicament is contained in the medicament cavity compared to a flow rate during inhalation if the medicament cavity is empty. As the inhalation flow also passes through the medicament cavity any content located therein effects the flow cross-section of the flow path along which the inhalation flow streams through the inhaler device. In particular, the flow cross-section of the flow path decreases due to the content in the medicament cavity. Moreover, due to any content located in the medicament cavity the resistance which has to be overcome in order to generate an inhalation flow and to maintain the inhalation flow is increased. The decreased cross-section of the flow path as well as the increased resistance can be measured by the flow rate sensor. Moreover, use of a flow rate sensor allows measurement of flow characteristics of the inhalation flow, such as duration of the inhalation, peak flow rate, change of the flow rate over time and the volume of the inhalation flow.

In an embodiment of the invention the processing unit is configured to detect the presence and/or the amount of the medicament formulation in the medicament cavity. Preferably, the medicament formulation is provided in the medicament cavity in such a way that the light being emitted from the optical sensor is able to access the medicament cavity and is thus able to sense the presence and/or the amount of the medicament formulation in the cavity. In case a flow rate sensor is used, the resistance in order to establish an inhalation flow through the medicament cavity increases the bigger the amount of the medicament formulation disposed in the medicament cavity is. When a capsule is used as a reservoir for the medicament formulation, the capsule becomes lighter as the powdered medicament formulation exits the capsule and is entrained in the inhalation flow. Thus, by measuring the flow rate the presence and/or the amount of the medicament formulation in the capsule can be determined. Preferably, the processing unit is able to determine the amount of the medicament formulation which has been entrained in the inhalation flow during inhalation by comparing the amount of medicament formulation present in the medicament chamber before and after inhalation. Thus, the processing unit may determine the amount of medicament formulation inhaled by the patient.

According to the invention the medicament cavity of the inhaler device is an elongated capsule chamber extending along a longitudinal axis and having two opposing ends which each extend transverse to the longitudinalaxis and restrict the capsule chamber in a direction along the longitudinal axis. The capsule chamber is adapted to receive a capsule in which the medicament formulation is contained, wherein the capsule chamber is sized and arranged such that the capsule upon inhalation moves in the capsule chamber along the longitudinal axis back and forth between the opposing ends. The inhaler device comprises opening elements which are adapted to open the capsule such that upon inhalation by the patient a medicament formulation in the capsule is entrained in the inhalation flow. Opening of the capsule may be achieved by the capsule being moved relative to one or more opening elements such as blades upon insertion of the capsule into the inhaler. Alternatively, opening elements which are moved relative to the capsule, e.g. subsequently to the capsule being inserted into the capsule chamber, may also be used. Preferably, the capsule is opened at both of its opposing end portions in order to allow the capsule being fully emptied during inhalation.

Preferably, the processing unit is further configured to detect the movement of the capsule in the capsule chamber and/or whether the capsule has been opened. Due to the movement of the capsule in the medicament chamber the inhalation flow measured by the flow rate sensor is not constant but fluctuating and comprises multiple peaks which are caused by the capsule impacting against the walls of the medicament chamber. Optionally, variation of the weight of the capsule and thus whether the capsule has been opened and the medicament formulation exits the capsule during inhalation can also be detected. Lighter capsules move faster compared to heavier capsules and thus impact more often onto the walls of the capsule chamber which can be detected by analyzing the flow rate of the inhalation flow.

According to the invention the air inlet duct of the inhaler device in flow direction of the inhalation flow is divided into a first intermediate duct and a second intermediate duct, wherein the first intermediate duct is connected to one of the ends of the capsule chamber and the second intermediate duct is connected to the other end of the capsule chamber. This arrangement allows the capsule to move back and forth in an oscillating fashion along the longitudinal axis thereby impacting at the two opposing ends of the capsule chamber. Upon impacting against the opposing ends the medicament formulation contained in the capsule is shaken and deagglomorated which simplifies the entrainment of the medicament formulation into the inhalation flow. The design of such an inhaler, its functioning and the oscillating movement of the capsule upon inhalation is for example disclosed in international patent application WO 2017/068171 A1.

According to an example which does not fall under the scope of the present invention, the primary sensor device comprises a flow rate sensor which is positioned along a flow path of the inhalation flow or is positioned in a bypass duct. No matter which of the above positions is chosen the flow rate sensor has to be positioned such that an effect on the inhalation flow by the sensor is minimized and at the same time reliable measurement of the flow rate of the inhalation flow is guaranteed. For example, the flow rate sensor may be positions in a bypass bypassing at least the medicament cavity. Alternatively, the bypass may bypass at least a portion of the first or second intermediate duct.

According to the invention the primary sensor device comprises a flow rate sensor which is positioned in the first intermediate duct or the second intermediate duct of the inhaler device in proximity to the air inlet duct. Upon inhalation the inhalation flow passes either the first intermediate duct or the second intermediate duct thereby moving the capsule in the capsule chamber until the capsule impacts on one of the opposing ends and closes the corresponding intermediate duct through which the inhalation flow passes. As the inhalation flow through the corresponding intermediate duct is omitted the inhalation flow passes through the other of the first and second intermediate ducts thereby generating an oscillating movement of the capsule. Consequently, upon inhalation and upon movement of the capsule in the capsule chamber the inhalation flow passes through the first and second intermediate duct in an alternating fashion depending on the position of the capsule. This relation is explained in detail in international patent application WO 2017/068171 A1 as referenced above. By positioning the flow rate sensor in one of the first and second intermediate ducts in proximity to the air inlet duct alternating flow in the first and second intermediate ducts can be analyzed as well as the flow rate of the inhalation flow in total. Thus, by using only one flow rate sensor the presence of a capsule in the capsule chamber as well as its movement during inhalation together with the flow rate of the inhalation flow in total can be detected.

In another embodiment of the invention the primary sensor device comprises multiple flow rate sensors, wherein a first flow rate sensor is positioned in one of the first and second intermediate ducts and a second flow rate sensor is positioned in the other of the first and second intermediate ducts or the second flow rate sensor is positioned in the air inlet duct. Multiple flow rate sensor increase the accuracy of the measurements taken. Moreover the reliability of the measurements is increased in such a way that even if one sensor fails measurements can still be taken by the remaining working sensor.

In another embodiment the primary sensor device further comprises an optical sensor which is positioned in proximity to the medicament cavity such that the optical sensor is capable of omitting light through the medicament cavity. Preferably, the optical sensor is positioned in a wall surrounding the medicament cavity.

In another embodiment the inhaler device further comprises a secondary sensor device and the processing unit is configured to detect the preparatory acts for preparing the inhaler device for inhalation based on sensor signals generated by the secondary sensor device. Preferably, the electronic system allows detection of the preparatory acts needed in order to prepare the inhaler device for inhalation as well as the inhalation of the medicament formulation itself. In connection with the present invention the term "preparatory acts" is understood as the acts being performed in order to set up the inhaler for inhalation. These acts may include amongst others opening of a cap covering the mouthpiece, insertion of a capsule or a dose of the medicament formulation in the medicament cavity and opening of the capsule before inhalation. However, the processing unit may also be configured to detect acts performed by the patient subsequent to the inhalation such as closing of the cap based on sensor signals generated by the at least one secondary sensor device.

In an embodiment of the invention the secondary sensor device of the inhaler device comprises a cap detection sensor for detecting whether a cap of the inhaler device covers or uncovers the mouth piece. Opening of the cap of the mouthpiece is one of the first steps needed in order to prepare the inhaler device for inhalation. Detecting the opening of the cap may also be used as a signal in order to wake up the electronics of the electronic system such as an electronic unit or the processing unit. Accordingly, the inhaler device may remain in a power saving mode during non-use and may be transmitted by opening of the cap of the mouthpiece into an active mode during which all sensors and electronics are activated in order to detect and analyze inhalation by a patient. The cap detection sensor may be for example a switch which is positioned in such a way that the switch is triggered when the cap is removed and/or when the cap is installed on the housing of the inhaler device.

In another embodiment the opening elements are piercing elements which are configured to protrude through the opposing ends of the capsule chamber in order to pierce the medicament capsule located therein, wherein the secondary sensor device comprises a piercing sensor which is adapted to sense a movement of the piercing elements when piercing the capsule. Such a piercing sensor may for example be a hall sensor which detects movement of the piercing elements into the capsule chamber. Use of a secondary sensor device in order to sense movement of the piercing elements is a more precise method in order to detect whether the capsule has already been opened. Thus, reliability of the piercing detection is increased.

Preferably, the processing unit is adapted to compare the sensor signals and/or the communicated data with reference values and to generate a feedback regarding the quality of inhaler use to the patient. The feedback regarding quality of inhaler use may contain feedback whether all the preparational acts such as opening of the cap of the mouth piece, insertion of a capsule, piercing of the capsule and inhalation have been accomplished and/or whether these preparational acts have been performed within a given time limit before the inhalation. The feedback regarding quality of inhaler use may also contain feedback regarding the acts performed by the patient subsequent to the inhalation such as closing of the cap. Additionally, the feedback regarding quality of inhaler use may also comprise feedback given with regard to the duration, speed and volume of the inhalation which shall be understood in connection with the present invention as the quality of inhalation. Moreover, the feedback regarding quality of inhaler use may also comprise feedback with regard to the amount of a medicament formulation being inhaled. Thus, due to the feedback regarding quality of inhaler use intake of the medicament can be analyzed and improved. The reference values for generation of the feedback regarding inhaler use may be stored on a memory located on the inhaler or the external device. Alternatively the reference values may also be stored on a server which is accessible by the external device and/or the inhaler device. Feedback may be given in an acoustic, visual or haptic way. Optionally, the inhaler device and/ or the external device may give instructions to the patient such as the sequence of preparatory acts as well as the sequence of inhalation steps (e.g. inhaling and exhaling). These instructions may also include instructions regarding the timing of the preparatory acts and/or the inhalation steps. The instructions may be giving in an acoustic, visual or haptic way.

In an embodiment of the invention, the processing unit is located on the inhaler device or the external device. If the processing unit is located on the inhaler device the inhaler device may be configured as a standalone device which is able to generate feedback to the patient without use of an additional external device. If the processing unit is located on the external device the inhaler device is configured to transmit data to the external device which then analyzes and evaluates the communicated data. The external device may be for example a smart phone, a hand held device or a personal computer and may be configured to provide acoustic, visual or haptical feedback.

The above object is also achieved by a method of using an electronic system as described above. The method comprises the steps of:
a) Providing the electronic system, wherein the primary sensor device comprises at least one flow rate sensor;
b) Exhaling into the inhaler device such that an expiratory flow is generated which flows through the air inlet duct towards the outlet duct or vice versa or into an add-on device fixed to the inhaler device;
c) Measuring the expiratory flow by the at least one flow rate sensor;
d) Processing and evaluating the sensor signals of the at least one flow rate sensor by the processing unit.

The inhaler device according to the present invention has been developed in order to be also used for spirometry. Spirometry is used in asthma and COPD therapy in order to determine the patent's lung volume and the change of the patient's lung volume during a corresponding therapy. Thus by integrating inhalation functions and spirometry functions within one single inhaler device, asthma and COPD treatment is simplified. There is no need for further devices in order to take spirometry measurements. Preferably the patient exhales into the air inlet duct or the outlet duct of the inhaler device or into an exhalation duct of the add-on device. Optionally the add-on device is removably fixed to the inhaler device. The at least one flow rate sensor of the inhaler device may be connected to the add-on device such that the flow rate sensor may take measurements of an exhalation flow in the add-on device. Preferably, the flow rate sensor may comprise a sensor probe which is located in the exhalation duct of the add-on device.

In an embodiment the expiratory flow in method step b) flows through the air inlet duct and the first and second intermediate ducts towards the outlet duct or vice versa. Preferably, the flow cross-sections of the inhaler device have been adapted such that the inhaler may be used for spirometry. Preferably the inhaler device comprises a bypass duct, which is optionally connected to the air inlet duct and the outlet duct and which at least bypasses the medicament cavity. Thus, the expiratory flow optionally flows from the air inlet duct into the bypass duct, thereby bypassing at least the medicament cavity, towards the outlet duct or vice versa.

Also disclosed is that the medicament cavity is not contaminated in method step b) by moisture in the expiratory flow. As the expiratory flow simultaneously passes through the first and second intermediate ducts a portion of the medicament cavity, in particular of the capsule chamber, in between its opposing ends is not flooded by the exhalation flow. Thus, moisture of the exhaled air does not stick to the walls of the medicament cavity. Consequently, clumping of the medical formulation after the inhaler has been used for spirometry is omitted. Optionally, in case a bypass duct is used in the inhaler device, at least the medicament cavity is not contaminated by the moisture in the expiratory flow. The expiratory flow bypasses at least the medicament cavity via the bypass duct. In case an add-on device is used, the expiratory flow streams through the exhalation duct of the add-on device. As the exhalation duct is separated from the inhaler device the inhaler device is not contaminated with moisture contained in the expiratory of the patient.

### BRIEF DESCRIPTION OF THE FIGURES

The invention will now be described in connection with an exemplary embodiment shown in the figures in which:
- Figure 1: shows an exploded view of the inhaler device according to the present invention and
- Figure 2: shows a cross-sectional view of the inhaler device according to Figure 1.

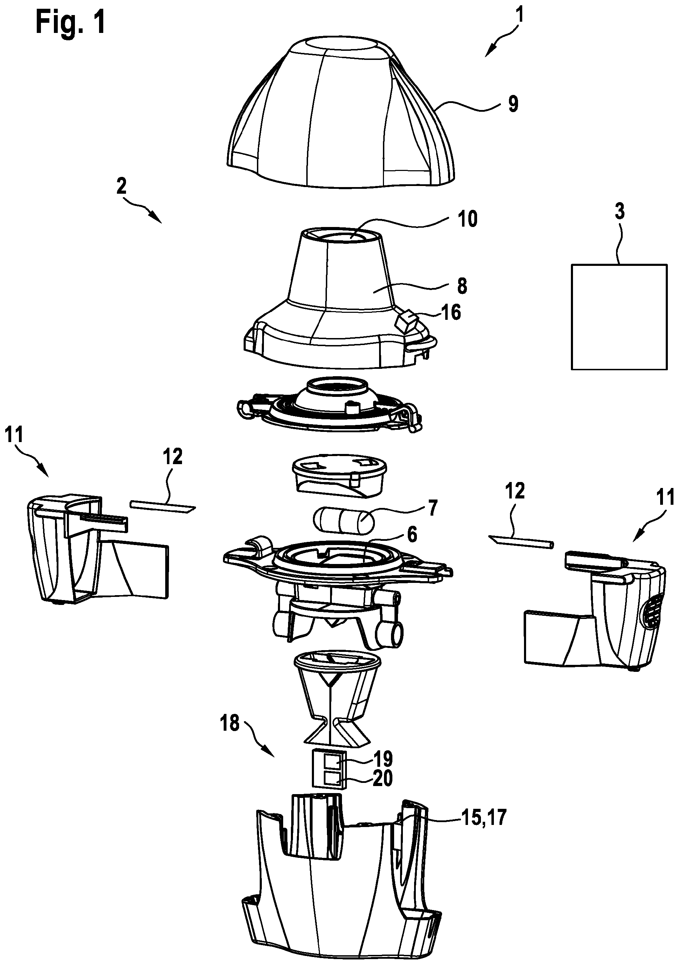

Figure 1 shows an electronic system 1 comprising an inhaler device 2 which is formed as a dry powder inhaler (DPI) for dispensing a medicament formulation in dry powder form. The electronic system 1 further comprises an external device 3 which is configured to receive data communicated from the inhaler device 2. The external device 3 may be a smart phone, a computer, a server or any other computational device.

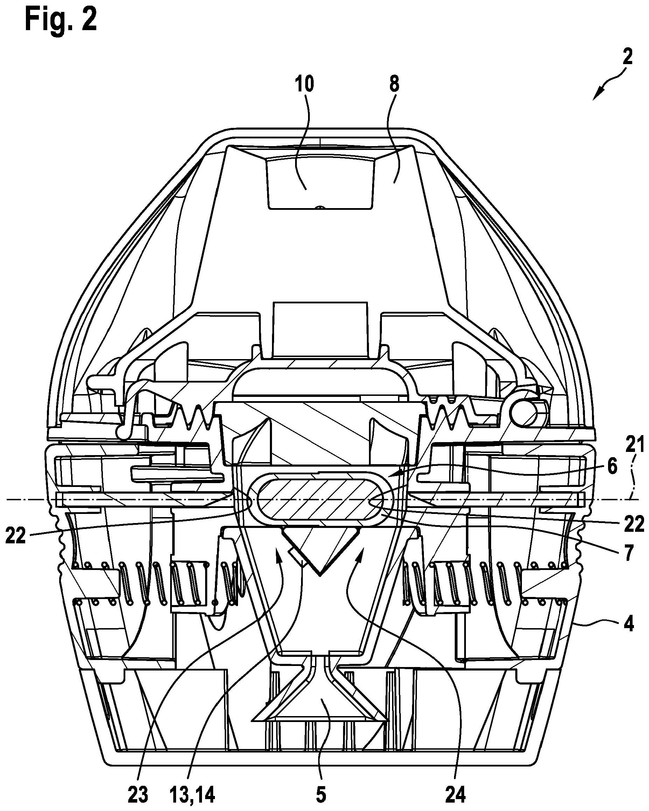

The inhaler device 2 comprises a housing 4 with an air inlet duct 5 (see Fig. 2) and a medicament cavity which is formed as an elongated capsule chamber 6. The capsule chamber 6 is connected to the air inlet duct 5 and is adapted to receive a capsule 7 in which the medicament formulation is contained.

The inhaler device 2 further comprises a mouthpiece 8 and a removable cap 9 which is adapted to cover the mouthpiece 8. The mouthpiece 8 has an outlet duct 10 which is connected to the capsule chamber 6 such that a patient upon inhalation through the outlet duct 10 causes an inhalation flow directed from the air inlet duct 5 through the capsule chamber 6 towards the outlet duct 10.

The inhaler device 2 further comprises opening elements formed as piercing elements 11. The piercing elements 11 comprise needles 12 which are adapted to open the capsule 7 such that upon inhalation by the patent the medicament formulation in the capsule 7 in entrained in the inhalation flow.

The inhaler device 2 further comprises at least one primary sensor device 13 which comprises a flow rate sensor 14 (see Fig. 2) and which is configured to generated sensor signals. The flow rate sensor 14 is formed as a differential pressure sensor. Additionally the primary sensor device 13 may comprise an optical sensor (not shown). If an optical sensor is used the sensor is positioned in proximity to the capsule chamber 6 such that the optical sensor is capable of emitting light through the capsule chamber 6.

The inhaler device 2 further comprises a secondary sensor device 15 configured to generate sensor signals. The secondary sensor device 15 comprises a cap detection sensor 16 such as a switch for detecting whether the cap 9 of the inhaler device 2 covers or uncovers the mouthpiece 8. Additionally, the secondary sensor device 15 comprises a piercing sensor 17 which is adapted to sense the movement of the piercing elements 11 when piercing the capsule 7. The piercing sensor 17 is formed as a hall sensor located in the housing 4, in particular proximity of the piercing elements 11.

The inhaler device further comprises an electronic unit 18 having a wireless transmitting unit 19 for communicating data related to the sensor signals. The electronic unit 18 is stored in the housing 4 of the inhaler device 2.

The electronic system 1 further comprises a processing unit 20 for processing the sensor signals from the primary sensor device 13, the secondary sensor device 15 and/or the communicated data. The processing unit 20 is located in the inhaler device 2 and is in particular part of the electronic unit 18. Alternatively, the processing unit is located on the external device 3.

The processing unit 20 is configured to analyze the content of the capsule chamber 6 based on the generated sensor signals by the primary sensor device 13. In particular the processing unit 20 is configured to detect the presence and/or the amount of the medicament formulation in the capsule chamber 6. Additionally, the processing unit 20 is further configured to detect movement of the capsule 7 in the capsule chamber 6. Moreover, the processing unit 20 is configured to detect preparatory acts for preparing the inhaler device 2 for inhalation based on the sensor signals generated by the secondary sensor device 15.

The processing unit 20 is adapted to compare the sensor signals from the primary and secondary sensor devices 13, 15 and/or the communicated data with reference values. Based on this comparison the processing unit 20 is configured to generate a feedback regarding the quality of inhaler use to the patient. The reference values may be stored in the electronic unit 18, on the external device 3 and/or a remote computational device which may be accessed by the inhaler device or the external device 3. The remote computational device may be for example a server. Optionally, the inhaler device 2 and/ or the external device 3 may give instructions to the patient such as the sequence of preparatory acts as well as the sequence of inhalation steps (e.g. inhaling and exhaling). These instructions may also include instructions regarding the timing of the preparatory acts and/or the inhalation steps. The instructions may be giving in an acoustic, visual or haptic way.

Figure 2 shows a cross sectional view of the inhaler device 2. The elongated capsule chamber 6 extends along a longitudinal axis 21 and has two opposing ends 22 which each extend transverse to the longitudinal axis 21 and restrict the capsule chamber 6 in a direction along the longitudinal axis 21.

The capsule chamber 6 is sized and arranged such that the capsule 7 upon inhalation moves in the capsule chamber 6 along the longitudinal axis 21 back and forth between the opposing ends 22. The piercing elements 11, in particular the needles 12 thereof (Figure 1), are configured to protrude through the opposing ends 22 of the capsule chamber 6 in order to pierce the medicament capsule 7 located therein. The piercing elements 11 are moveable relative to the housing 4 along the longitudinal axis 21 of the capsule chamber 6. Upon protrusion of the needles 12 of the piercing elements 11 through the opposing ends 22 of the capsule chamber 6 the capsule 7 is opened such that upon inhalation by the patient the medicament formulation and the capsule 15 is entrained in the inhalation flow.

The air inlet duct 5 of the inhaler device 2 is in flow direction of the inhalation flow divided into a first intermediate duct 23 and a second intermediate duct 24, wherein the first intermediate duct 23 is connected to one of the ends 22 of the capsule chamber 6 and the second intermediate duct 24 is connected to the other end 22 of the capsule chamber 6. A detailed description of the intermediate duct, the capsule chamber and the movement of the capsule upon inhalation is disclosed in international patent application WO 2017/068171 A1.

The flow rate sensor 14 is positioned along a flow path of the inhalation flow. In particular the flow rate sensor 14 is positioned in the first intermediate duct 23 of the inhaler device in proximity to the air inlet duct 5, in particular at the division of the first intermediate duct 23 and the second intermediate duct 24.

Alternatively, the inhaler device 2 comprises multiple flow rate sensors 14, wherein a first flow rate sensor is positioned in one of the first and second intermediate ducts 23, 24 and a second flow rate sensor is positioned in the other of the first and second intermediate ducts 23, 24 or the second flow rate sensor is positioned in the air inlet duct 5. According to the present invention the electronic system 1 is also adapted to be used for spirometry. A corresponding method will be explained in the following:
In a first step the electronic system 1 is provided to the patient, wherein the primary sensor device comprises at least one flow rate sensor. In a second step the patient exhales into the air inlet duct 5 of the inhaler device 2 such that an expiratory flow is generated which flows through the air inlet duct 5 and the first and second intermediate ducts 23, 24 towards the outlet duct 10. In a third step the expiratory flow is measured by the at least flow rate sensor 14. In a fifth step the sensor signals generated by the flow rate sensor 14 are processed in evaluated by the processing unit 20 which is either stored in the inhaler device 2 or the external device 3.

During the exhalation of the patient into the air inlet duct the capsule chamber 6 is not contaminated by moisture in the expiratory flow. This is because the exhalation flow simultaneously flows through the first and second intermediate ducts 23, 24 and thus bypasses a middle portion of the capsule chamber 6 located in between the opposing ends 22 of the capsule chamber 6.

### Reference numerals

- 1: electronic system
- 2: inhaler device
- 3: external device
- 4: housing
- 5: air inlet duct
- 6: capsule chamber (medicament cavity)
- 7: capsule
- 8: mouth piece
- 9: cap
- 10: outlet duct
- 11: piercing elements
- 12: needles
- 13: primary sensor device
- 14: flow rate sensor
- 15: secondary sensor device
- 16: cap detection sensor
- 17: piercing sensor
- 18: electronic unit
- 19: wireless transmitting unit
- 20: processing unit
- 21: longitudinal axis (capsule chamber)
- 22: opposing ends (capsule chamber)
- 23: first intermediate duct
- 24: second intermediate duct

## Claims

1. Electronic system comprising:
an inhaler device (2) for dispensing a medicament formulation in dry powder form and
an external device (3) which is configured to receive data communicated from the inhaler device (2),
wherein the inhaler device (2) comprises:
a housing (4) with an air inlet duct (5);
a medicament cavity (6) which is connected to the air inlet duct (5) and which is configured to hold at least a dose of the medicament formulation, and
a mouthpiece (8) having an outlet duct (10) which is connected to the medicament cavity (6) such that a patient upon inhalation through the outlet duct (10) causes an inhalation flow directed from the air inlet duct (5) through the medicament cavity (6) towards the outlet duct (10), wherein the inhaler device (2) further comprises a primary sensor device (13) which is configured to generate sensor signals and a wireless transmitting unit (19) for communicating data related to the sensor signals, and
wherein the electronic system (1) further comprises a processing unit (20) for processing the sensor signals and/or the communicated data,
wherein the processing unit (20) is configured to analyze the content of the medicament cavity (6) based on the generated sensor signals of the primary sensor device (13), and
wherein the medicament cavity of the inhaler device is an elongated capsule chamber (6), the capsule chamber (6) being adapted to receive a capsule (7) in which the medicament formulation is contained, wherein the capsule chamber (6) is sized and arranged such that the capsule (7) upon inhalation moves in the capsule chamber (6), wherein the inhaler device (2) comprises opening elements (11) which are adapted to open the capsule (7) such that upon inhalation by the patient the medicament formulation in the capsule (7) is entrained in the inhalation flow,
**characterized in**
**that** the capsule chamber (6) extends along a longitudinal axis (21) and has two opposing ends (22) which each extend transverse to the longitudinal axis (21) and restrict the capsule chamber (6) in a direction along the longitudinal axis (21), wherein the capsule chamber (6) is sized and arranged such that the capsule (7) upon inhalation moves in the capsule chamber (6) along the longitudinal axis (21) back and forth between the opposing ends (22), that the air inlet duct (5) of the inhaler device (2) in flow direction of the inhalation flow is divided into a first intermediate duct (23) and a second intermediate duct (24), wherein the first intermediate duct (23) is connected to one of the ends (22) of the capsule chamber (6) and the second intermediate duct (24) is connected to the other end (22) of the capsule chamber (6), that the primary sensor device (13) comprises a flow rate sensor (14) which is positioned in the first intermediate duct (23) or the second intermediate duct (24) of the inhaler device (2) in proximity to the air inlet duct (5).

2. Electronic system according to claim 1, **characterized in that** the primary sensor device (13) further comprises one or more of an optical sensor, a flow rate sensor (14), a touch sensor, a proximity sensor or any combination thereof.

3. Electronic system according to any of the preceding claims **characterized in that** the processing unit (20) is configured to detect the presence and/or the amount of the medicament formulation in the medicament cavity (6).

4. Electronic system according to claim 1, **characterized in that** the processing unit (20) is further configured to detect the movement of the capsule (7) in the capsule chamber (6) and/or whether the capsule (7) has been opened.

5. Electronic system according to claim 1, **characterized in that** the primary sensor device (13) comprises multiple flow rate sensors (14), wherein a first flow rate sensor (14) is positioned in one of the first and second intermediate ducts (23, 24) and a second flow rate sensor (14) is positioned in the other of the first and second intermediate ducts (23, 24) or the second flow rate sensor (14) is positioned in the air inlet duct (5).

6. Electronic system according to any of the preceding claims, **characterized in that** the primary sensor device (13) further comprises an optical sensor which is positioned in proximity to the medicament cavity (6) such that the optical sensor is capable of emitting light through the medicament cavity (6).

7. Electronic system according to any of the preceding claims, **characterized in that** the inhaler device (2) further comprises a secondary sensor device (15) and that the processing unit (20) is configured to detect preparatory acts for preparing the inhaler device (2) for inhalation based on sensor signals generated by the secondary sensor device (15).

8. Electronic system according to claim 7, **characterized in that** the secondary sensor device (15) of the inhaler device (2) comprises a cap detection sensor (16) for detecting whether a cap (9) of the inhaler device (2) covers or uncovers the mouthpiece (8).

9. Electronic system according to claim 7, **characterized in that** the opening elements are piercing elements (11) which are configured to protrude through the opposing ends (22) of the capsule chamber (6) in order to pierce the medicament capsule (7) located therein, wherein the secondary sensor device (15) comprise a piercing sensor (17) which is adapted to sense the movement of the piercing elements (11) when piercing the capsule (7).

10. Electronic system according to any of the preceding claims, **characterized in that** the processing unit (20) is adapted to compare the sensor signals and/or the communicated data with reference values and to generate a feedback regarding the quality of inhaler use to the patient.

11. Electronic system according to any of the preceding claims, **characterized in that** the processing unit (20) is located on the inhaler device (2) or the external device (3) .

12. Method of using an electronic system according to any of the claims 1 to 11 for spirometry, the method comprising the steps of:
a) Providing the electronic system (1), wherein the primary sensor device (13) comprises at least one flow rate sensor (14);
b) Exhaling into the inhaler device (2) such that an expiratory flow is generated which flows through the air inlet duct (5) towards the outlet duct (10) or vice versa;
c) Measuring the expiratory flow by the at least one flow rate sensor (14);
d) Processing and evaluating the sensor signals of the at least one flow rate sensor (14) by the processing unit (20) .

13. Method according to claim 12, wherein the expiratory flow in step b) flows through the air inlet duct (5) and the first and second intermediate ducts (23, 24) towards the outlet duct (10) or vice versa.

## Patentansprüche

1. Elektronisches System, umfassend:
eine Inhalationsvorrichtung (2) zur Abgabe einer Medikamentenformulierung in Trockenpulverform und
eine externe Vorrichtung (3), die so konfiguriert ist,
dass sie von der Inhalationsvorrichtung (2) übermittelte Daten empfängt,
wobei die Inhalationsvorrichtung (2) umfasst:
ein Gehäuse (4) mit einem Lufteinlasskanal (5);
einen Medikamentenhohlraum (6), der mit dem Lufteinlasskanal (5) verbunden ist und der so konfiguriert ist, dass er mindestens eine Dosis der Medikamentenformulierung enthält, und
ein Mundstück (8) mit einem Auslasskanal (10), der mit dem Medikamentenhohlraum (6) verbunden ist, so dass ein Patient bei Inhalation durch den Auslasskanal (10) einen Inhalationsstrom verursacht, der von dem Lufteinlasskanal (5) durch den Medikamentenhohlraum (6) zu dem Auslasskanal (10) gerichtet ist,
wobei die Inhalationsvorrichtung (2) weiterhin eine primäre Sensorvorrichtung (13), die zum Erzeugen von Sensorsignalen konfiguriert ist, und eine drahtlose Sendeeinheit (19) zum Übermitteln von Daten in Bezug auf die Sensorsignale umfasst, und
wobei das elektronische System (1) weiterhin eine Verarbeitungseinheit (20) zur Verarbeitung der Sensorsignale und/oder der übermittelten Daten umfasst,
wobei die Verarbeitungseinheit (20) ausgebildet ist, den Inhalt des Medikamentenhohlraums (6) basierend auf den erzeugten Sensorsignalen der primären Sensorvorrichtung (13) zu analysieren, und
wobei der Medikamentenhohlraum der Inhalationsvorrichtung eine längliche Kapselkammer (6) ist, wobei die Kapselkammer (6) angepasst ist, eine Kapsel (7) aufzunehmen, in der die Medikamentenformulierung enthalten ist, wobei die Kapselkammer (6) so bemessen und angeordnet ist, dass sich die Kapsel (7) beim Inhalieren in der Kapselkammer (6) bewegt, wobei die Inhalationsvorrichtung (2) Öffnungselemente (11) umfasst, die geeignet sind, die Kapsel (7) zu öffnen, so dass bei Inhalation durch den Patienten die Medikamentenformulierung in der Kapsel (7) in den Inhalationsstrom mitgenommen wird,
**dadurch gekennzeichnet,**
**dass** sich die Kapselkammer (6) entlang einer Längsachse (21) erstreckt und zwei gegenüberliegende Enden (22) aufweist, die sich jeweils quer zur Längsachse (21) erstrecken und die Kapselkammer (6) in einer Richtung entlang der Längsachse (21) begrenzen, wobei die Kapselkammer (6) so bemessen und angeordnet ist, dass sich die Kapsel (7) bei Inhalation in der Kapselkammer (6) entlang der Längsachse (21) zwischen den gegenüberliegenden Enden (22) hin und her bewegt,
**dass** der Lufteinlasskanal (5) der Inhalationsvorrichtung (2) in Strömungsrichtung des Inhalationsstroms in einen ersten Zwischenkanal (23) und einen zweiten Zwischenkanal (24) unterteilt ist, wobei der erste Zwischenkanal (23) mit einem der Enden (22) der Kapselkammer (6) und der zweite Zwischenkanal (24) mit dem anderen Ende (22) der Kapselkammer (6) verbunden ist,
**dass** die primäre Sensorvorrichtung (13) einen Durchflusssensor (14) umfasst, der in dem ersten Zwischenkanal (23) oder dem zweiten Zwischenkanal (24) der Inhalationsvorrichtung (2) in der Nähe des Lufteinlasskanals (5) angeordnet ist.

2. Elektronisches System nach Anspruch 1, **dadurch gekennzeichnet, dass** die primäre Sensorvorrichtung (13) weiterhin einen oder mehrere optische Sensoren, Durchflusssensoren (14), Berührungssensoren, Näherungssensoren oder eine beliebige Kombination davon umfasst.

3. Elektronisches System nach einem der vorhergehenden Ansprüche, **dadurch gekennzeichnet, dass** die Verarbeitungseinheit (20) ausgebildet ist, das Vorhandensein und/oder die Menge der Medikamentenformulierung in dem Medikamentenhohlraum (6) zu erfassen.

4. Elektronisches System nach Anspruch 1, **dadurch gekennzeichnet, dass** die Verarbeitungseinheit (20) ferner dazu ausgebildet ist, die Bewegung der Kapsel (7) in der Kapselkammer (6) zu erfassen und/oder zu erkennen, ob die Kapsel (7) geöffnet wurde.

5. Elektronisches System nach Anspruch 1, **dadurch gekennzeichnet, dass** die primäre Sensorvorrichtung (13) mehrere Durchflusssensoren (14) umfasst, wobei ein erster Durchflusssensor (14) in einem der ersten und zweiten Zwischenkanäle (23, 24) und ein zweiter Durchflusssensor (14) in dem anderen der ersten und zweiten Zwischenkanäle (23, 24) oder der zweite Durchflusssensor (14) in dem Lufteinlasskanal (5) angeordnet ist.

6. Elektronisches System nach einem der vorhergehenden Ansprüche, **dadurch gekennzeichnet, dass** die primäre Sensorvorrichtung (13) weiter einen optischen Sensor umfasst, der in der Nähe des Medikamentenhohlraums (6) positioniert ist, so dass der optische Sensor in der Lage ist, Licht durch den Medikamentenhohlraum (6) zu emittieren.

7. Elektronisches System nach einem der vorangehenden Ansprüche, **dadurch gekennzeichnet, dass** die Inhalationsvorrichtung (2) ferner eine sekundäre Sensorvorrichtung (15) umfasst und, dass die Verarbeitungseinheit (20) ausgebildet ist, vorbereitende Handlungen zur Vorbereitung der Inhalationsvorrichtung (2) für die Inhalation basierend auf Sensorsignalen zu erkennen, die von der sekundäre Sensorvorrichtung (15) erzeugt wurden.

8. Elektronisches System nach Anspruch 7, **dadurch gekennzeichnet, dass** die sekundäre Sensorvorrichtung (15) der Inhalationsvorrichtung (2) einen Kappendetektionssensor (16) umfasst, um zu detektieren, ob eine Kappe (9) der Inhalationsvorrichtung (2) das Mundstück (8) abdeckt oder freilegt.

9. Elektronisches System nach Anspruch 7, **dadurch gekennzeichnet, dass** die Öffnungselemente Durchstechelemente (11) sind, die ausgebildet sind, durch die gegenüberliegenden Enden (22) der Kapselkammer (6) hindurchzuragen, um die darin befindliche Medikamentenkapsel (7) zu durchstechen, wobei die sekundäre Sensorvorrichtung (15) einen Durchstechsensor (17) umfasst, der ausgelegt ist, die Bewegung der Durchstechelemente (11) beim Durchstechen der Kapsel (7) zu erfassen.

10. Elektronisches System nach einem der vorhergehenden Ansprüche, **dadurch gekennzeichnet, dass** die Verarbeitungseinheit (20) dazu eingerichtet ist, die Sensorsignale und/oder die übermittelten Daten mit Referenzwerten zu vergleichen und eine Rückmeldung über die Qualität der Inhalatorbenutzung an den Patienten zu erzeugen.

11. Elektronisches System nach einem der vorhergehenden Ansprüche, **dadurch gekennzeichnet, dass** die Verarbeitungseinheit (20) auf der Inhalationsvorrichtung (2) oder der externen Vorrichtung (3) angeordnet ist.

12. Verfahren zur Verwendung eines elektronischen Systems nach einem der Ansprüche 1 bis 11 zur Spirometrie, wobei das Verfahren die folgenden Schritte umfasst:
a) Bereitstellen des elektronischen Systems (1), wobei die primäre Sensorvorrichtung (13) mindestens einen Durchflusssensor (14) umfasst;
b) Ausatmen in die Inhalationsvorrichtung (2), so dass ein exspiratorischer Fluss erzeugt wird, der durch den Lufteinlasskanal (5) in Richtung des Auslasskanals (10) oder umgekehrt fließt;
c) Messen des Expirationsflusses durch den mindestens einen Durchflusssensor (14);
d) Verarbeiten und Auswerten der Sensorsignale des mindestens einen Durchflusssensor (14) durch die Verarbeitungseinheit (20).

13. Verfahren nach Anspruch 12, wobei der Exspirationsstrom in Schritt b) durch den Lufteinlasskanal (5) und den ersten und zweiten Zwischenkanal (23, 24) in Richtung des Auslasskanals (10) oder umgekehrt fließt.

## Revendications

1. Système électronique comprenant :
un inhalateur (2) pour distribuer une formulation médicamenteuse sous forme de poudre sèche et
un dispositif externe (3) qui est configuré pour recevoir des données communiquées par l'inhalateur (2),
dans lequel l'inhalateur (2) comprend :
un logement (4) avec un tube d'entrée d'air (5) ;
une cavité à médicament (6) qui est reliée au tube d'entrée d'air (5) et qui est configurée pour retenir au moins une dose de formulation médicamenteuse, et
un embout buccal (8) ayant un tube de sortie (10) qui est relié à la cavité à médicament (6) de telle façon qu'un patient, à l'inhalation à travers le tube de sortie (10), fait qu'un flux d'inhalation soit dirigé du tube d'entrée d'air (5) à travers la cavité à médicament (6) en direction du tube de sortie (10),
dans lequel l'inhalateur (2) comprend en outre un dispositif de capteur principal (13) qui est configuré pour générer des signaux de capteur et une unité de transmission sans fil (19) pour communiquer des données liées aux signaux de capteur, et
dans lequel le système électronique (1) comprend en outre une unité de traitement (20) pour traiter les signaux de capteur et/ou les données communiquées, dans lequel l'unité de traitement (20) est configurée pour analyser le contenu de la cavité à médicament (6) en se basant sur les signaux de capteur générés du dispositif de capteur principal (13), et
dans lequel la cavité à médicament de l'inhalateur est une chambre de capsule (6) allongée, la chambre de capsule (6) étant adaptée pour recevoir une capsule (7) dans laquelle la formulation médicamenteuse est contenue, dans lequel la chambre de capsule (6) est dimensionnée et arrangée de façon à ce que la capsule (7), lors de l'inhalation, se déplace dans la chambre de capsule (6), dans lequel l'inhalateur (2) comprend des éléments d'ouverture (11) qui sont adaptés pour ouvrir la capsule (7) de façon à ce qu'à l'inhalation par le patient, la formulation médicamenteuse dans la capsule (7) soit entraînée dans le flux d'inhalation,
**caractérisé en ce que**
la chambre de capsule (6) s'étend le long d'un axe longitudinal (21) et présente deux extrémités opposées (22) qui s'étendent chacune transversalement à l'axe longitudinal (21) et restreignent la chambre à capsule (6) dans une direction le long de l'axe longitudinal (21), dans lequel la chambre de capsule (6) est dimensionnée et agencée de telle façon que la capsule (7), à l'inhalation, se déplace dans la chambre de capsule (6) le long de l'axe longitudinal (21) d'avant en arrière entre les extrémités opposées (22),
que le tube d'entrée d'air (5) de l'inhalateur (2) dans la direction d'écoulement du flux d'inhalation est divisé en un premier tube intermédiaire (23) et un second tube intermédiaire (24), dans lequel le premier tube intermédiaire (23) est relié à l'une des extrémités (22) de la chambre de capsule (6) et le second tube intermédiaire (24) est relié à l'autre extrémité (22) de la chambre de capsule (6), que le dispositif de capteur principal (13) comprend un capteur de débit (14) qui est positionné dans le premier tube intermédiaire (23) ou le second tube intermédiaire (24) de l'inhalateur (2) à proximité du tube d'entrée d'air (5).

2. Système électronique selon la revendication 1, **caractérisé en ce que** le dispositif de capteur principal (13) comprend en outre un ou plusieurs d'un capteur optique, d'un capteur de débit (14), d'un capteur tactile, d'un capteur de proximité ou toute combinaison de ceux-ci.

3. Système électronique selon l'une quelconque des revendications précédentes, **caractérisé en ce que** l'unité de traitement (20) est configurée pour détecter la présence et/ou la quantité de la formulation médicamenteuse dans la cavité de médicament (6).

4. Système électronique selon la revendication 1, **caractérisé en ce que** l'unité de traitement (20) est en outre configurée pour détecter le mouvement de la capsule (7) dans la chambre de capsule (6) et/ou si la capsule (7) a été ouverte.

5. Système électronique selon la revendication 1, **caractérisé en ce que** le dispositif de capteur principal (13) comprend plusieurs capteurs de débit (14), dans lequel un premier capteur de débit (14) est positionné dans l'un des premier et second tubes intermédiaires (23, 24), et un second capteur de débit (14) est positionné dans l'autre des premier et second tubes intermédiaires (23, 24) ou bien le second capteur de débit (14) est positionné dans le tube d'entrée d'air (5).

6. Système électronique selon l'une quelconque des revendications précédentes, **caractérisé en ce que** le dispositif de capteur principal (13) comprend en outre un capteur optique qui est positionné à proximité de la cavité de médicament (6) de façon à ce que le capteur optique soit capable d'émettre de la lumière à travers la cavité de médicament (6).

7. Système électronique selon l'une quelconque des revendications précédentes, **caractérisé en ce que** l'inhalateur (2) comprend en outre un dispositif de capteur secondaire (15) et que l'unité de traitement (20) est configurée pour détecter des actes préparatoires pour préparer l'inhalateur (2) pour l'inhalation en se basant sur des signaux de capteur générés par le dispositif de capteur secondaire (15).

8. Système électronique selon la revendication 7, **caractérisé en ce que** le dispositif de capteur secondaire (15) de l'inhalateur (2) comprend un capteur de détection de bouchon (16) pour détecter si un bouchon (9) de l'inhalateur (2) couvre ou découvre l'embout buccal (8).

9. Système électronique selon la revendication 7, **caractérisé en ce que** les éléments d'ouverture sont des éléments perçants (11) qui sont configurés pour faire saillie à travers les extrémités opposées (22) de la chambre de capsule (6) afin de percer la capsule de médicament (7) située à l'intérieur, dans lequel le dispositif de capteur secondaire (15) comprend un capteur perçant (17) qui est adapté pour détecter le mouvement des éléments perçants (11) lors du perçage de la capsule (7).

10. Système électronique selon l'une quelconque des revendications précédentes, **caractérisé en ce que** l'unité de traitement (20) est adaptée pour comparer les signaux de capteurs et/ou les données communiquées à des valeurs de référence et pour générer un retour concernant la qualité de l'utilisation de l'inhalateur au patient.

11. Système électronique selon l'une quelconque des revendications précédentes, **caractérisé en ce que** l'unité de traitement (20) est située sur l'inhalateur (2) ou sur le dispositif externe (3).

12. Procédé d'utilisation d'un système électronique selon l'une quelconque des revendications 1 à 11 pour la spirométrie, le procédé comprenant les étapes de :
a) fournir le système électronique (1), dans lequel le dispositif de capteur principal (13) comprend au moins un capteur de débit (14) ;
b) expirer dans l'inhalateur (2) de façon à ce qu'un flux expiratoire soit généré qui s'écoule à travers le tube d'entrée d'air (5) en direction du tube de sortie (10) ou vice versa ;
c) mesurer le flux expiratoire par l'au moins un capteur de débit (14) ;
d) traiter et évaluer les signaux de capteur de l'au moins un capteur de débit (14) par l'unité de traitement (20).

13. Procédé selon la revendication 12, dans lequel le flux expiratoire à l'étape b) circule à travers le tube d'entrée d'air (5) et les premier et second tubes intermédiaires (23, 24) en direction du tube de sortie (10) ou vice versa.
